(19) Europäisches Patentamt
European Patent Office
Office européen des brevets

(11) Publication number: **0 480 843 A2**

## (12) EUROPEAN PATENT APPLICATION

(21) Application number: **91402730.5**

(22) Date of filing: **11.10.91**

(51) Int. Cl.5: **A61L 33/00**

(30) Priority: **11.10.90 JP 270424/90**

(43) Date of publication of application:
**15.04.92 Bulletin 92/16**

(84) Designated Contracting States:
**BE DE FR GB IT NL SE**

(71) Applicant: **TERUMO KABUSHIKI KAISHA
No. 44-1, Hatagaya 2-chome, Shibuya-ku
Tokyo 151(JP)**

(72) Inventor: **Takano, Yoshihito
c/o Terumo K. K., 1500, Inokuchi,
Nakai-machi
Ashigarakami-gun, Kanagawa-ken(JP)**
Inventor: **Nagai, Hiroshi
c/o Terumo K. K., 1500, Inokuchi,
Nakai-machi
Ashigarakami-gun, Kanagawa-ken(JP)**
Inventor: **Takeda, Eiko
c/o Terumo K. K., 1500, Inokuchi,
Nakai-machi
Ashigarakami-gun, Kanagawa-ken(JP)**

(74) Representative: **Gillard, Marie-Louise et al
Cabinet Beau de Loménie 55, Rue
d'Amsterdam
F-75008 Paris(FR)**

(54) Antithrombogenic medical material and medical device.

(57) An antithrombogenic medical material having attached to the surface of a substrate such as polymeric material a peptide containing an amino acid lysine residue at the C terminal thereof, wherein the peptide is a hydrolyzate of fibrin, and the material is used for an artificial organ.

BACKGROUND OF THE INVENTION

Field of the Invention:

This invention relates to a novel antithrombogenic material and a medical device. More particularly, it relates to a novel antithrombogenic medical material adapted at least to contact blood and to a medical device having the surface thereof for contact with blood formed of the antithrombogenic medical material.

Description of the Prior Art:

In consequence of the tremendous advance made in recent years in medical treatments, various medical devices such as, for example, artificial blood vessels, vascular catheters, extracorporeal circuits for blood circulation, and artificial organs which by nature are used at sites directly exposed to blood have come to find extensive utility. These medical devices are required only naturally to possess ideal physical properties including durability and to exhibit outstanding antithrombogenisity and histocompatibility as well.

As means for imparting antithrombogenisity to medical devices, the following methods have been adopted.

(1) A method which utilizes the construction and physical properties of the material itself for the medical device in enabling this medical device to manifest antithrombogenisity.

(2) A method which utilizes the antithrombogenisity of end otherial cells.

(3) A method which comprises attaching such an antithrombogenic substance as an anticoagulant to the part of a medical device destined to contact with blood to give the antithrombogenisity.

In the field of artificial blood vessels, an artificial blood vessel made of a porous polytetrafluoroethylene may be cited as an example of the method of (1). This artificial blood vessel of porous polytetrafluoroethylene utilizes the inactivity of this material to blood in causing the blood vessel to exhibit an antithrombogenisity.

An artificial blood vessel made of porous polyester may be cited as an embodiment of the method of (2). This artificial blood vessel of porous polyester utilizes its ability of tissue incorporation and neointima formation, early formation of neointima and consequently manifesting an antithrombogenisity.

In the case of an artificial blood vessel made of porous polytetrafluoroethylene, however, the restoration of tissues after transplantation tends to be delayed in a great measure because the material itself is inactive to the vital body. In other words, because tissues are hard to invade into the pores of this artificial blood vessel, this artificial blood vessel encounters the disadvantage that the formation of neointima occurs only with difficulty, the organization of tissues is retarded, the connection between the tissues formed on the inner wall and the outer wall thereof is established insufficiently, and the inoculated part tends to form pannus.

Then, in the case of an artificial blood vessel made of porous polyester, when this blood vessel has a large inside diameter, it forms a surface structure similarly to the natural blood vessel and consequently manifests an antithrombogenisity owing to the fact that thrombi adhere to the inner wall of the blood vessel immediately after transplantation and a neoplastic internal membrane covers the deposited thrombi. When the artificial blood vessel has a small inside diameter, however, it has a problem of complete clogging of the vessel with thrombi which are deposited on the inner wall thereof immediately after transplantation.

As a measure which promises solution of these problems, the method of (3) has come to attract special attention. The substances which are currently studied for practicability in this method include such proteins as collagen and fibrin which originate in vital tissues and such thrombin activity depressing factors as heparin, for example.

Particularly, the fibrin is known to activate a t-PA (tissue plasminogen activating factor) which is an enzyme acting on the blood plasminogen system (Collen. D.: "Thrombos Haemostas. 43, 77, 1980). In other words, the fibrin indirectly manifests an antithrombogenisity by activating the t-PA.

An object of this invention, therefore, is to provide a novel antithrombogenic medical material and a medical device produced by molding the antithrombogenic medical material in a stated shape.

SUMMARY OF THE INVENTION

The object described above is accomplished by an antithrombogenic medical material characterized by having attached to the surface of a substrate a peptide possessing an amino acid lysine residue at a C terminal thereof, wherein the peptide is a hydrolyzate of fibrin.

The object described above is further accomplished by a medical device which has at least the surface thereof for contact with blood formed with the antithrombogenic medical material mentioned above.

BRIEF DESCRIPTION OF THE DRAWINGS

Fig. 1 is a diagram showing the relation between the activity of a t-PA of a plasmin-treated fibrin according with the present invention and the time of the plasmin treatment,
Fig. 2 is a diagram showing the relation between the amount of amino acids (lysine residues and arginine residues) produced by the plasmin treatment and the time of the plasmin treatment,
Fig. 3 is a diagram showing the relation between the t-PA activities severally of the plasmin-treated fibrin deprived of a lysine residue by the carboxypeptidase B treatment and not deprived of a lysine residue by the treatment and the time of the plasmin treatment, and
Fig.4 is a diagram showing the relation between an absorbance and concentration of protein.

EXPLANATION OF THE PREFERRED EMBODIMENT

The present invention will be described in detail below.
Though there are many peptides originated from various proteins possessing an amino lysine residue at the C terminal thereof, fibrin proves to be ideal in point of biocompatibility and availability. The peptide will be described hereinbelow, therefore, with reference to the enzymatic hydrolyzate of fibrin as a typical example. It should be noted, however, that this invention is not limited to the enzymatic hydrolyzates of fibrin but is applied to all the peptides which have an amino acid lysine residue at the C terminal thereof. In the present invention, the term "peptide" is used in the broad sense enough to embrace even fragmental peptides.
The fibrin is formed by restriction decomposing fibrinogen by the action of thrombin thereby effecting release of fibrinopeptides A and B. To be more specific, the fibrinogen is hydrolyzed by thrombin and, with release of fibrinopeptide A (FPA) from the A-alpha chain and fibrinopeptide B (FPB) from the B-beta chain in all the component peptide chains, converted consequently into fibrin monomers. The fibrin monomers are aggregated and polymerized into an unstable fibrin polymer (soluble fibrin polymer). The fibrin polymer is further hydrolyzed with thrombin and the gamma chains intervening between the fibrin molecules are cross-linked (through an isopeptide bond between a lysine residue and a glutamine residue) with an activated factor XIII to give rise to a firm stable fibrin polymer (insoluble fibrin polymer).
By hydrolyzing this fibrin with a proteinase and inducing appearance of an amino acid lysine residue at the C terminal therefore, a peptide having the antithrombogenic property conspicuously improved as compared with an untreated fibrin and having the biocompatibility retained intact can be produced. Though the amount of the amino acid lysine residue to be thus produced is not particularly restricted, it is ideally in the range of from 2 to 20 m.mols, preferably from 5 to 10 m.mols, per mg of the fibrin. The amount of the amino acid lysine residue to be produced can be approximately determined by treating a peptide possessing the lysine residue with enzyme such as carboxypeptidase to expel and release the lysine residue therefrom and measuring the amount of the release lysine residue.
As the enzyme to be used for this hydrolysis of fibrin, the plasmin is ideally used. The plasmin is an enzyme which exhibits high substrate specificity to fibrin and fibrinogen in particular and hydrolyzes the carbonyl side of a lysine or arginine residue in the protein.
The antithrombogenic medical device of this invention is produced by depositing on the surface of a substrate a peptide having a lysine residue produced as described above.
The substrate has no particular restriction except for the requirement that it should avoid exerting any adverse effect on blood. For example, the materials which are favorably usable for artificial organs, particularly artificial blood vessels, include polyurethane, urethane-urea copolymers, blends of these polymers or copolymers with silicone type polymers, polymeric materials such as polyesters and polytetrafluoroethylene, and other well-known materials for artificial blood vessels. In this case, the polyurethane type polymers and urethane-urea copolymers are preferable to be of the polyether type in point of durability in the vital system. Particularly, polyether-segmentized polyurethane and polyether-segmentized polyurethane urea and the like prove to be preferable.
These polymeric materials, when necessary, may incorporate therein by blending a stabilizer, a plasticizer, and a lubricant, for example.
The material for the medical device may be selected, depending on the use for which the medical device is intended and on the kind itself of the medical device, from among polyvinyl chloride, polyethylene, polypropylene, nylon, ethylene-vinyl acetate copolymer, polyvinyl chloride-polyurethane copolymer, polyvinyl chloride-(ethylene-vinyl acetate copolymer) copolymer, silicone rubber, polypropylene, polycarbonate, high-density polyethylene, and acrylic resin, for example.

The material for the medical device is preferable to possess a porous texture in point of biocompatibility (high incorporation of tissues) and ease of peptide deposition.

The attachment of the fibrin hydrolyzate, for example, to the surface of the substrate described above is attained ideally by a method which comprises immersing the substrate in a fibrinogen solution, removing it from the solution, then immersing the substrate in a thrombin solution thereby forming a fibrin gel thereon, and causing the fibrin gel-covered substrate to contact an enzyme-containing solution thereby effecting hydrolysis of the fibrin gel. The order of the solutions in which the substrate is to be sequentially immersed is not particularly limited to that which has been indicated in the procedure described above. Optionally, the attachment may be accomplished by separately forming the fibril gel in advance and subsequently applying it to the substrate.

In addition to the above-mentioned process, as a method for giving the above-mentioned fibrin hydrolyzate on such substrate, there are, for example, various known processes such as physicochemical process by dipping the substrate into a solution of the fibrin hydrolyzate and irradiating plasma or radiation, a chemical cross-linking process using glutaraldehyde or carbodimide, and a method wherein a cross-linkable resin prepolymer, e.g., a photocross-linkable resin prepolymer or a urethane resin prepolymer is kneaded previously with the substrate and the kneaded mixture is reacted with fibrin hydrolyzate. Further, there is a process wherein fibrin hydrolyzate is applied on the substrate and then the substrate is contacted with an enzyme-containing solution to hydrolyze.

For the purpose of enhancing the attachability of peptide to the surface of the substrate, the surface of the substrate may be treated with an acid, plasma, or ozone, for example, and consequently enabled to acquire hydrophilicity.

Incidentally, the peptide which has been attached to the surface of the substrate is generally preferably to defy separation from the surface for a long time after transplantation. Occasionally, the attachability of peptide may be controlled so as to adjust the time at which the peptide is preferable to be released from the surface.

The medical device according with the present invention is produced by forming at least the surface thereof destined to contact blood with the antithrombogenic medical material. Typical examples of the medical device of this description are various artificial organs represented by artificial blood vessels and artificial organs such as artificial hearts, artificial kidneys, and artificial lungs. The medical device can be applied severally to the main bodies of these organs or to parts of these organs such as, for example, gas-exchange membranes and dializing membranes.

Particularly, the artificial blood vessels not only of the grade having a relatively large inside diameter but also of the grade having a small inside diameter of even below 5 mm can be produced with an outstanding antithrombogenisity.

The medical device of this invention can be further applied to various tubes which make up an extracorporeal circuit for blood circulation, connectors for joining tubes, catheters for insertion into the veins and the arteries, bubble traps, blood bags, chambers, and centrifugal pumps, for example.

The medical device of this invention can also be applied to catheters for retention in the blood vessels and devices for transfusion and collection of blood such as syringes, blood collecting tubes, blood bags, and accessories thereof, for example.

Now, the present invention will be described more specifically hereinbelow with reference to working examples.

Example 1

(1) Determination of plasmin-treated fibrin decomposition products ability to enhance activity of t-AP:

A human fibrinogen preparation (produced by Kabi Corp. and marketed under trademark designation of "Vitrum, grade L") was dissolved in distilled water. The resultant solution was applied to columns packed with Gelatin-Cellulofine (proprietary product of Seikagaku Kogyo K.K.) and Lysine-Sepharose (proprietary product of Pharmacia) for the removal of fibronectin and plasminogen. A 50 mM phosphate buffer (pH 7.5) was used for eluent. The concentration of the eluted fibrinogen solution was determined by the coagulation time measuring method using Data-Fi (proprietary product of Dade Corp.).

The fibrinogen solution was adjusted with a 50 mM Hepes buffer solution (pH 7.4) containing 20 mM $CaCl_2$, 2 mM $MgCl_2$, and 150 mM NaCl to a concentration of 2 mg/ml and dispensed in a unit volume of 200 $\mu l$ among 24 wells in a tissue petri dish. To each wells 10 $\mu l$ of a 20 units/ml thrombin solution (produced by Mochida Seiyaku K.K.) were added and the dish was incubated overnight at 37°C to produce

4

fibrin gel (untreated fibrin). Then, the fibrin gels were treated with 0.5 ml of a 0.05 unit/ml plasmin at 37°C for stepped durations of 0, 5, 15, 30, and 60 minutes, then added each with 1000 KIE/ml of Trasyrol ® to neutralize plasmin. The gels were washed three to four times with PBS, to obtain plasmin-treated fibrin decomposition products.

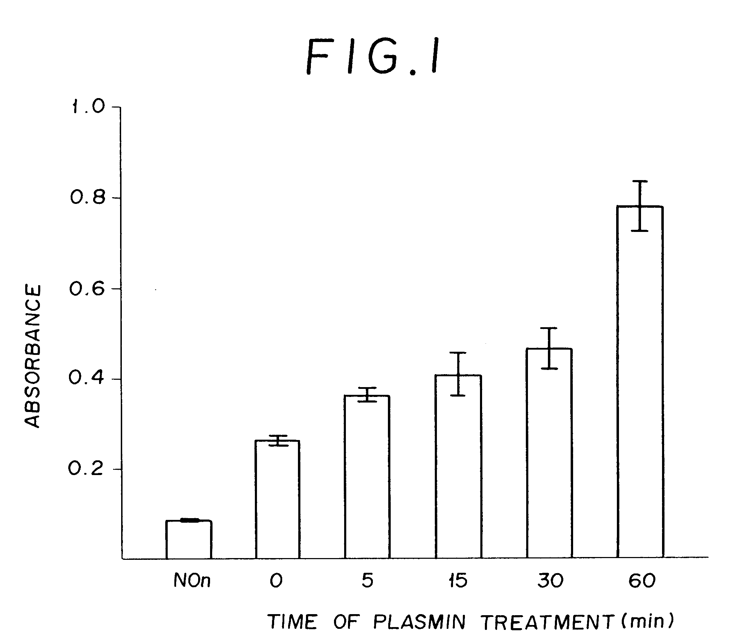

The untreated fibrin and the plasmin-treated fibrin decomposition products consequently obtained were each combined with 200 ul of a 0.08 mg/ml Glu-Plasminogen (proprietary product of Biopool Corp.) adjusted in advance with a 50 mM Tris-HCl buffer (pH 8.8), 200 $\mu$l of a 1.74 mM H-D-Valyl-L-leucyl-L-lysine-P-nitroanilide dihydrochloride (colo-producing substrate) (S-2251; Daiichi Kagaku Yakuhin K.K.), and 400 $\mu$l of a 80 unit/ml t-PA (produced by Biopool Corp.), incubated at 37°C for about 20 minutes, inactivated to cease the reaction in process by the addition of 1 ml of a 2% citric acid and measured the absorbance at 405 nm. The results are shown in Fig. 1.

It is clearly noted from Fig. 1 that the presence of the untreated fibrin (indicated as "Non" in the diagram) enhanced the activity of the t-PA and the plasmin-treated fibrin decomposition products (working example of this invention) enhanced the activity of the t-PA conspicuously as compared with the untreated fibrin.

(2) Analysis of plasmin-treated fibrin for amino acid at the C terminal:

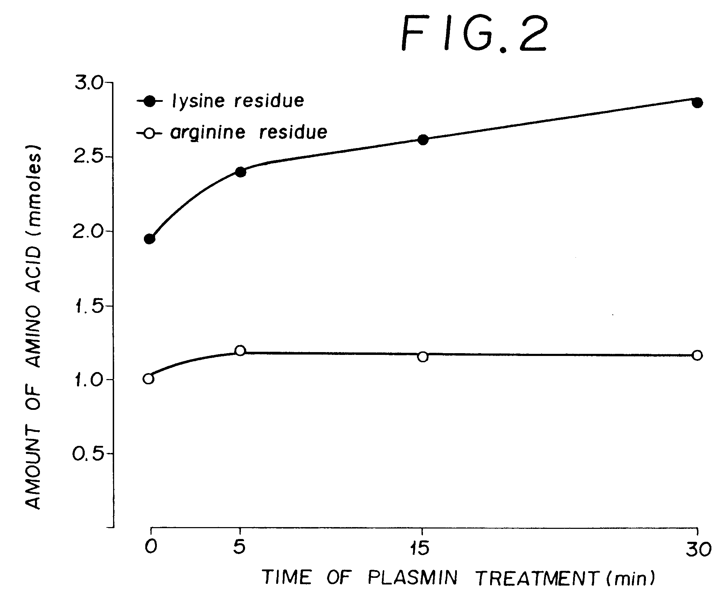

Fibrin gels were prepared in 24 wells of a tissue incubation petri dish by following the procedure of (1) above, each combined with 0.5 ml of a 0.05 unit/ml plasmin, and treated at 37°C for stepped durations of 0, 5, 15, and 30 minutes. The gels were thoroughly washed with PBS, and then treated with a 25 units/ml carboxypeptidase B at 37°C for 30 minutes. The supernatants which consequently formed in the wells were analyzed for amino acid residues with an amino acid analyzer (produced by Shimadzu Seisakusho K.K.). The results are shown in Fig. 2.

It is clearly noted from Fig. 2 that the treatment of fibrin with plasmin brought about appearance of an arginine residues and a lysine residues and that the amount of the lysine residue increased in accordance as the duration of the treatment with plasmin elongated. It is also clear from the data that the lysine residue formed by the treatment with plasmin as described above was eliminated by the treatment with the carboxypeptidase B.

(3) Effect of the presence of lysine residue on enhancement of the activity of t-PA:

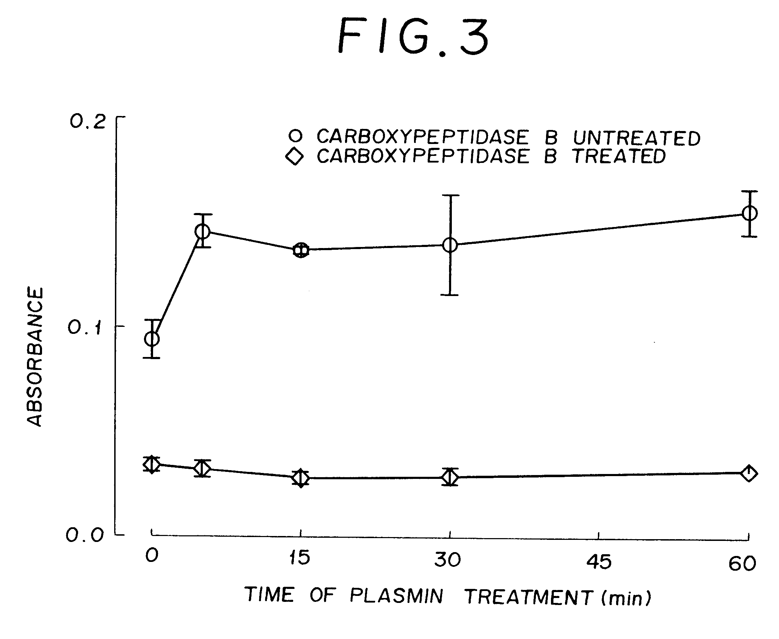

Fibrin gels were prepared by following the procedure of (1) mentioned above and treated with a 0.05 unit/ml plasmin at 37°C for stepped durations of 0, 15, 30, 45, and 60 minutes. They were subjected to the treatment with the carboxypeptidase B in the same manner as in (2) above. The plasmin-treated fibrin were tested for ability to enhance the activity of the t-PA in the same manner as in (1) above. The results are shown in Fig. 3.

It is clearly noted from Fig. 3 that the samples which had undergone the treatment with the carboxypeptidase B were deprived of the lysine residue at the C terminal and, therefore, showed conspicuous decreases in the ability to enhance the activity of the t-PA as compared with the untreated samples.

(4) Effect of lysine residue on antithrombogemisity:

A tube of polyester measuring 4 mm in inside diameter and 11 cm in overall length was immersed in a 30 mg/ml fibrinogen solution (produced by Kabi Corp. and marketed under trademark designation of "Vitrum grade L"). After a while, the tube was removed from the solution and immersed in a 15 units/ml thrombin solution to induce attachment of fibril gel to the inner wall surface of the tube. A rod 3.8 mm in diameter was inserted through the tube to smoothen the inner wall surface of the tube. The tube was incubated overnight at 37°C. Subsequently, the tube was immersed in a 0.05 unit/ml plasmin solution, treated therein at 37°C for 15 minutes, and immersed and neutralized in a 200 units/ml Trazirol, and thereafter thoroughly washed with a phosphate buffer, to afford an artificial blood vessel A (working example of the present invention). An artificial blood vessel B (control) was obtained by separately preparing an artificial blood vessel A, immersing this blood vessel A in a 25 units/ml carboxypeptidase B, treating it at 37°C for 30 minutes, and thoroughly washing it with a phosphate buffer.

The artificial blood vessels A or the artificial blood vessels B was connected via I. V. catheter (produced by Terumo Kabushiki Kaisha and marketed under trademark designation of "Surflow 14G") to the carotid artery and the jugular vein of a rabbit and put to a 24-hour blood circulation test. The time-course changes in the number of blood platelets found by the test are shown in Table 1. The test used two rabbits per sample.

Table 1

| Sample | Change in number of blood platelets (%) (mean ± standard deviation) | | | |
|---|---|---|---|---|
| | Initial | After 60 min. | After 180 min. | After 24 hrs. |
| Artificial | 100 | 77.6 ± 12.9 | 74.8 ± 8.4 | 52.1 ± 1.6 |
| Artificial blood vessel B | 100 | 102.8 ± 11.6 | 75.9 ± 17.5 | 7.8 ± 0.6 |

Example 2

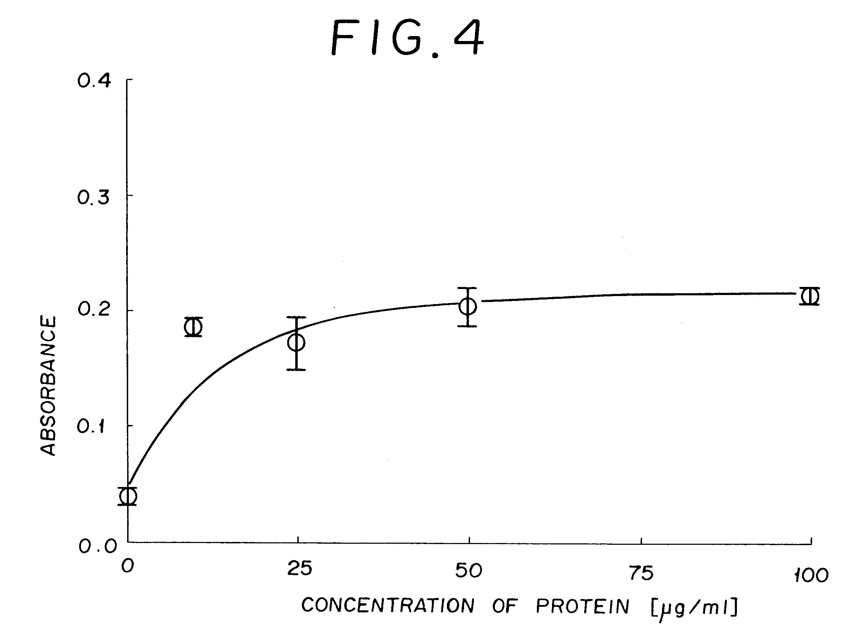

Into fibrin obtained by a method of (1) in Example 1, 0.5 ml of 0.5 U/ml of plasmin was added and incubated at a temperature of 37 °C for one night. The solution thus formed was added to microplates for ELISA in an amount of each 100 $\mu$l in concentrations of 10-100 $\mu$g/ml and stood for one night. After washing, activity of the t-PA was measured by the same method as (1) in Example 1 except that 1/10 scale was used. The results were shown in Fig.4.

It is clearly noted from Table 1 that in the test run using the artificial blood vessel B (control) having attached thereto the plasmin-treated fibrin deprived of the lysine residue, 24 hours' blood circulation brought about a conspicuous decrease in the number of blood platelets. In contrast, in the test run using the artificial blood vessel A (working example of this invention) coated with the plasmin-treated fibrin decomposition product retaining the lysin residue, no appreciable decrease was recognized in the number of blood platelets. When the inner wall surfaces of the artificial blood vessel A and the artificial blood vessel B were visually examined after 24 hours' blood circulation, absolutely no adhesion of red thrombi was recognized on the inner wall surface of the artificial blood vessel A and conspicuous attachment of red and white blood thrombi was recognized on the inner wall surface of the artificial blood vessel B.

The antithrombogenic medical material of the present invention possesses outstanding antithrombogenisity and cell inducing property because it is characterized by having attached to the surface of a substrate a peptide containing an amino acid lysine residue at the C terminal thereof.

## Claims

1. An antithrombogenic medical material having attached to the surface of a substrate a peptide containing an amino acid lysine residue at the C terminal thereof, wherein said peptide is a hydrolyzate of fibrin.

2. A medical material according to claim 1, wherein said hydrolyzate is an enzymatic hydrolyzate.

3. A medical material according to claim 2, wherein said enzyme is a proteinase.

4. A medical material according to claim 3, wherein said proteinase is plasmin.

5. A medical material according to claim 1, wherein the amount of said amino acid lysine residue of said peptide to be produced is in the range of from 2 to 20 m.mols per mg of said fibrin.

6. A medical material according to claim 1, wherein said substrate is a polymeric material.

7. A medical material according to claim 6, wherein said polymeric material is a porous material.

8. A medical device having at least the surface thereof for contact with blood formed with an antithrombogenic medical material having attached to the surface of a substrate a peptide containing an amino acid lysine residue at the C terminal thereof.

9. A medical device according to claim 8, wherein said peptide is the hydrolyzate of fibrin.

10. A medical device according to claim 8, wherein said substrate is a polymeric material.

11. A medical device according to claim 8, which is an artificial organ.

FIG.1

FIG.2

AMOUNT OF AMINO ACID (mmoles)

lysine residue
arginine residue

TIME OF PLASMIN TREATMENT (min)

EP 0 480 843 A2

# FIG.3

O CARBOXYPEPTIDASE B UNTREATED
◇ CARBOXYPEPTIDASE B TREATED

ABSORBANCE

0.2

0.1

0.0

0    15    30    45    60

TIME OF PLASMIN TREATMENT (min)

EP 0 480 843 A2

FIG.4